Europäisches Patentamt

European Patent Office

Office européen des brevets

(19)

(11) Publication number: **0 449 878 B1**

## EUROPEAN PATENT SPECIFICATION

(12)

(45) Date of publication of patent specification: **24.05.95**

(51) Int. Cl.⁶: **A61L 27/00**, A61F 2/32, C08L 23/06

(21) Application number: **90900461.6**

(22) Date of filing: **28.11.89**

(86) International application number: **PCT/US89/05262**

(87) International publication number: **WO 90/06140 (14.06.90 90/14)**

(54) **PROCESS OF MANUFACTURING ULTRAHIGH MOLECULAR WEIGHT LINEAR POLYETHYLENE SHAPED ARTICLES.**

(30) Priority: **02.12.88 US 278912**
**24.12.88 US 288576**
**24.10.89 US 426918**

(43) Date of publication of application:
**09.10.91 Bulletin 91/41**

(45) Publication of the grant of the patent:
**24.05.95 Bulletin 95/21**

(84) Designated Contracting States:
**AT BE CH DE ES FR GB IT LI LU NL SE**

(56) References cited:

**Polymer, Volume 22, January 1981, IPC Business Press, (London, GB), S.K. BHATEJA: "Unaxial Tensile Creep Behaviour of Ultra High Molecular Weight Linear Polyethylene", page 23-28**

**Journal of Polymer Science: Part A2, Polymer Physics, Volume 7, 1969, John Wiley & Sons, (New York, US), T.D. DAVIDSON et al.:**

**"Extended-Chain Chrystals. II. Crystallization of Polyethylene under Elevated Pressure", pages 2051-2059**

(73) Proprietor: **E.I. DU PONT DE NEMOURS AND COMPANY**
**1007 Market Street**
**Wilmington**
**Delaware 19898 (US)**

(72) Inventor: **HOWARD, Edward, George, Jr.**
**844 Old Public Road**
**Hockessin, DE 19707 (US)**
Inventor: **LI, Stephen**
**14 Servan Court**
**Wilmington, DE 19805 (US)**

(74) Representative: **Jones, Alan John et al**
**CARPMAELS & RANSFORD**
**43 Bloomsbury Square**
**London, WC1A 2RA (GB)**

EP 0 449 878 B1

**Description**

BACKGROUND OF THE INVENTION

1. Field of the Invention

This invention relates to a novel process for making ultrahigh molecular weight linear polyethylene (UHMWLPE). This novel UHMWLPE, in the form of a shaped article, exhibits a unique combination of properties making the material useful as a bearing surface, in general, but particularly useful as a prosthetic hip joint cup and as other prosthetic shapes for replacement of other joints of the human body. This article is the subject of copending European patent application 89312565.8 (Publication N° 0373800)

2. Description of the Prior Art

In U.S. Patent No. 3,944,536 (March 1976), Lupton et al describe UHMWPE in the form of a fabricated article exhibiting an elastic modulus of 340,000 to 500,000 psi, a tensile impact strength of 140 to 600 ft lb/in$^2$, a density of 0.95 to 0.98 g/cc at 25°C, a crystalline melting point of 142 to 148°C (as measured by differential thermal analysis) and a unique crystalline form characterized by the absence of fold spacings of 50-2000 Angstrom units (Å) and the presence of crystal spacings of about 10,000 Å. The critical feature of the process of producing this UHMWPE is disclosed to involve inducing crystallization of the molten polymer above 150°C by rapidly increasing the applied pressure from an initial level of 1 to 1000 atmospheres to a second level of 2000 to 7000 atmospheres and then cooling rapidly while maintaining a pressure sufficient to maintain the polyethylene in the solid phase until the temperature is below the crystalline melting point of the polyethylene at atmospheric pressure.

In Kunstuffe German Plastics 77 (1987) pp. 617-622, in an article entitled "Ultrahigh Molecular Polyethylene for Replacement Joints", Eyrer et al. point out that the service life of joint replacements made of UHMWPE is limited. Analysis of the damage to over 250 explanted hip cups and tibial plateaus revealed a changed property profile which they explained by post-crystallization resulting from oxidative chain decomposition. They suggested optimizing the processing of polyethylene under higher pressure and higher temperature to increase the degree of crystallinity. The Eyrer et al. product displays a creep of above 5% at a compression of 1000 psi (6.9 N/mm$^2$) for 24 hours at 37°C.

One of the most remarkable advances in the medical field in recent years is the development of prosthetic joints, particularly the load bearing hip. The crippled and sometimes bed ridden elderly can walk again. The key to this development is UHMWPE because, not only does it have the necessary impact strength, but it initiates no adverse blood reactions. But at present, these prosthetic joints are limited to the older, less active segment of the population because the polymer tends to creep under the pressure that a younger more active person might develop while involved in recreation or employment. The creep would cause the loss of the desired tolerance required between the plastic socket and the polished metal ball attached to the femur. These changes in dimensions disturb the distribution of walking forces which in turn accelerates more creep and wear. Eventually the increased pain requires a traumatic revision operation. One objective of this invention is to provide a process for making UHMWPE prosthetic joints with improved creep resistance hence removing some of the age restriction existing with regard to the present polyethylene joints.

SUMMARY OF THE INVENTION

The object of this invention is to provide a process for making a tough UHMWLPE composition and articles that display a creep resistance, when exposed to a temperature of 23±1°C and a relative humidity of 50±2% for 24 hours under a compression of 1000 psi, of less than 1% without sacrificing excellent tensile and flexural properties.

Specifically, the product obtained is a shaped UHMWLPE article exhibiting an elastic or flexural modulus of 250,000-500,000 psi, a tensile stress at yield of 3500-4500 psi, a tensile stress at break of 4000-9000 psi, a tensile modulus of 300,000-700,000 psi, an elongation of 200-500%, a notched Izod impact resistance of 12-20 ft. lb. per in. of notch, a creep at a compression of 1000 psi of less than 1% after 24 hours at a temperature of 23°C and a relative humidity of 50%, the polyethylene having a molecular weight of 400,000-10,000,000 (the molecular chain length between folds being greater than 3500Å), a single crystalline melting point of greater than 144°C (as measured by differential scanning calorimetry) the reduction in said melting point upon remelting being greater than 11°C above that of the starting polymer and an infrared crystallinity index of at least about 0.45.

The process for obtaining the shaped article of this invention involves six (6) important steps:

1. forming, by milling or casting or the like the article from UHMWLPE having a molecular weight of 400,000-10,000,000, preferably at least 1,000,000 and most preferably at least 6,000,000;

2. surrounding the article with an inert material that is collapsible and impermeable; and placing the surrounded article in a pressure vessel containing a gaseous fluid, preferably argon;

3. heating the vessel to a temperature of at least 190°C but no greater than 300°C, preferably 200°C-230°C, and raising the pressure in the vessel to at least 2800 atmospheres (ATM), preferably at least 3000 ATM;

4. maintaining the temperature and pressure substantially as selected in step 3 for at least 0.5 hour, preferably at least one hour;

5. thereafter, cooling by reducing the temperature to a temperature at least below about 160°C-170°C preferably to 160°C or below, most preferably below 140°C, while maintaining a pressure of at least 2800 ATM preferably at least 3000 ATM, at a slow rate, the rate of cooling being such that temperature gradients in the shaped article are substantially avoided. The polymer must be cooled slowly at the high pressure until it is fully crystallized. At 3000 ATM pressure, the crystallization temperature of UHMWLPE of over one million molecular weight is in the range of 170°C-190°C. The pressurized vessel should be cooled slowly to insure that the temperature of the polymer is not significantly above the vessel temperature, particularly if the pressure vessel construction does not permit means for measuring the temperature of the polymer itself; and

6. cooling and releasing the pressure on the shaped article in a manner such that any remelting of the article is prevented. This is accomplished by cooling at least to a temperature below the atmospheric pressure melting point, i.e., about 130°C-135°C preferably below 120°C, most preferably below 100°C and releasing the pressure to reduce it from at least 2800 ATM to approximately 1 ATM, either sequentially or simultaneously. It should be understood that it is necessary to cool the polymer to a temperature below its melting point at any particular pressure to insure that none of the polymer melts as the pressure is reduced since lowering the pressure lowers the melting point.

It has been found necessary to protect the surface of the article by enclosing it in a thin can during the process.

A very important step is the fifth step, i.e. cooling in a manner that limits severe temperature gradients in the article. For example, for a 1 inch X 6 inch rod, a cooling rate of approximately 10°C per hour is usually necessary. Cooling rates no greater than 10°C per hour are preferred. Whatever cooling rate is used, cooling requires careful control in order to limit temperature gradients during cooling. Cooling rapidly, as taught in the prior art, will not provide the desired article.

An additional step is expected to further improve the usefulness of the resulting product. A preliminary heat treatment is applied which subjects the UHMWPE to a temperature approaching, but not reaching, the decomposition point of the UHMWPE, preferably of between 320-340°C in an inert atmosphere for at least 0.5 hours.

This invention is particularly useful for manufacturing shaped articles where temperature gradients pose a problem during the cooling step, i.e., where the article's cross-sectional dimensions are at least 1 inch x at least 1 inch, usually for joints at least 1 inch x at least 2 inches. Specifically, the importance of this step and of this invention is manifest in producing articles having as its smallest dimension 0.2 inch, i.e., at least 0.2 inch in thickness. It has been found that in such articles, the temperature gradients must still be controlled by the process of this invention in order to obtain the desired product.

In addition to utility in the field of orthopedic replacement, the products are expected to prove useful in other applications also requiring the special properties of the products. Not only shaped articles are of interest, but also films and fibers as well as other "downstream" forms and unshaped granular forms of the products will prove useful. Film to be formed of the product of Example 1 is described in Example 6. These examples are illustrative only, and other forms, shaped and unshaped, of the composition are contemplated within the scope of the invention. Therefore, "article" shall include both shaped articles and unshaped articles.

In the best mode known at this time for using the process of this invention, the gas used in the pressure vessel is argon. Specifically, the shaped article is formed from commercially available UHMWLPE. It is necessary to protect the UHMWLPE from any entry of the gas into the polymer by surrounding the article completely with a thin stainless steel or similar metal can. It should be understood that other gaseous fluids may be used in place of argon. So long as the gas is not affected by the temperatures and pressures used in the process, the gas may be used. Such gases include, but are not lmited to, the noble gases, nitrogen, etc.

In the next step, the protected article is placed in an argon-filled pressure vessel and a pressure of at least 2000 ATM is applied with argon and the vessel is heated to about 220°C for about 6 hours. Thereafter, the temperature is "ramped" down at a rate no greater than about 10°C per hour to about 160°C while maintaining the pressure above 2800 ATM. The temperature is then "ramped" down at a maximum rate to 50°C while maintaining the high pressure, after which the pressure is released.

For purposes of this invention, ultrahigh molecular weight linear polyethylene (UHMWLPE) is defined as a linear polyethylene having an estimated weight-average molecular weight in excess of about 400,000, usually 1,000,000 to 10,000,000 as defined by a melt index (ASTMD-1238) of essentially zero and a reduced specific viscosity (RSV) greater than 8, preferably 25-30. The relationships of RSV to intrinsic viscosity and to molecular weight are those developed by R. Chaing as presented by P. S. Francis et al. in J. Polymer Science, 31, 453 (1958).

The improved properties of the products of this process are reflected in a tensile modulus of at least 300 kpsi, a flex modulus of at least 250 kpsi, ultimate tensile strength greater than 4000 psi, yield strength greater than 3500 psi and an elongation at break no greater than 500%.

A very important property of the product is its creep resistance. For prosthetic devices, e.g. knee, hip, elbow joints, etc., any substantial creep can be devastating in the loss of the benefits of extremely expensive surgery. Thus, the shaped articles resulting from this invention display as little as a 0.5% loss in thickness when subjected to a compression pressure of 1000 psi for 24 hours at a temperature of 23°C and a relative humidity of 50% in accordance with ASTM D-621.

Perhaps the most characteristic property of the product is its infrared crystallinity index (IRCI). This property, which provides a reasonably accurate reflection of the crystallinity of this material, is in a range never before attained with any polyethylene materials. To determine this index, samples are first obtained by microforming thin sections. Heat and pressure should be avoided during preparation of the samples. IRCI is the ratio of the band at 1894 reciprocal centimeters ($cm^{-1}$) to the band at 1305 reciprocal centimeters ($cm^{-1}$). Since the band at 1894 $cm^{-1}$ is attributed to the crystalline nature of the material and the band at 1305 $cm^{-1}$ is attributed to its amorphous nature, IRCI increases as the crystallinity increases. The product displays an IRCI of at least 0.45. In fact, values of 0.73 and higher have been obtained. On the other hand, IRCI values for prior known UHMWLPE's seldom reach above 0.3.

It should be appreciated that the step of forming the article by milling, casting, or the like from UHMWLPE may be performed as the first step in the process (i.e., before heating or preheating) or as the last step in the process (i.e., after the cooling step).

The invention will be more clearly understood by referring to the drawing and example, which follow. In the drawing, Figure 1 is a schematic diagram of the equipment used in the process for forming the product of the invention using argon gas.

In the example, most of the properties are measured using standard ASTM tests. All of the physical measurements were carried out under constant humidity (50% relative humidity) and temperature (23°C) conditions.

Tensile modulus, ultimate tensile strength, yield strength and elongation are measured according to ASTM D-638 with the following modifications:
- samples machined into shape without lubricating fluid
- type I tensile bar
- cross head speed = 0.2"/min for tensile modulus
    2.0"/min for tensile stress and elongation.

Creep resistance is measured in accordance with ASTM D-621 with the following modifications:
- samples machined into cylinders without the use of lubricating fluids
- samples measured 0.5" x 0.5" x 0.5"

Flexural properties are measured according to ASTM D-790 with the following modifications:
- samples machined into shape without the use of lubricating fluids
- typical flex bar measures 0.125" thick x 0.5" width x 5" length
- span or gage is 2.0". (This was determined by a span/depth ratio of 16/1.)
- cross head speed = 0.05"/min (calculated based on span).

Impact resistance is measured using the notched Izod test given in ASTM D-256 with the following modifications:
- samples machined into shape without the use of lubricating fluid
- type A or notched IZOD
- specimen size is 0.5" x 2.5"
- 0.4" from bottom of vertex to opposite side
- 1.25" impacted end (from end of bar to vertex of notch)

4

- the notch should be the specified angle of 22.5 degrees.

The following non-limiting examples illustrate the basic principles and unique advantages of the present invention. Various changes and modifications may be made without departing from the spirit and scope of the present invention.

EXAMPLE 1

The material used in this example is American Hoechst 415 GUR ultrahigh molecular weight polyethylene. It was obtained in the form of bars, 3" in diameter and up to 5' long in length. The material will be referred to as UHMWLPE. The molecular weight was over 1,000,000.

One or more pieces of the UHMWLPE 11 were placed into stainless steel, seamless, 48" long cylinders or sleeves 12. The thickness of the stainless steel was 1/8". The bottom of the cylinders was closed by welding a stainless steel cap 13 onto the bottom of the cylinder. The top of the cylinder was partially closed by welding on a modified cap 14 which contained a vacuum port not shown. The cylinder was then evacuated using a vacuum pump and sealed by crimping the port to form a can that surrounds the piece of UHMWLPE completely. The sealed cylinder was then placed in a containment vessel 16 large enough to hold 15 cylinders. The containment vessel 16 was then placed into a hot isostatic pressing (HIP) unit 17 with molybdenum heating units 18. Thermocouples were added to monitor the temperature of the cylinders.

The basic function of the HIP process is to uniformly heat a load while applying pressure uniformly to all surfaces. The pressure medium used in this case was argon. The gas entered at 15 and exited at 19. The UHMWPE is protected from the argon by the stainless steel cans.

The process conditions were:
1. Apply pressure to 39,000 psi.
2. Heat to 220°C.
3. Hold for 6 hours at 220°C and a minimum pressure of 41,000 psi.
4. Ramp temperature down at a rate no faster than 10°C per hour to 160°C. Pressure is maintained above 41,000 psi during this time.
5. Ramp temperature down at maximum rate to 50°C while maintaining the pressure above 41,000 psi.
6. Below 50°C, pressure may be let down and the cycle ended.

The UHMWPE rods were then removed from the sleeves and parts were fabricated for physical testing. It is noted that the material produced exhibits much higher tensile modulus, flex modulus, melting point, density and creep resistance than the starting material (Control A).

| Material | DSC Melting Point (°C) | Density (grams/cc) | IRCI |
|---|---|---|---|
| Control | 137.0-140.7°C | .93-.94 | 0.24 |
| Example 1 | 148.0-152.0°C | .947 | ≥ 0.45 |

| **ASTM D638** | **Control A** | **Example 1** |
|---|---|---|
| **Flex Modulus kpsi** | 165 | 291 |
| **Tensile Modulus kpsi** | 185 | 315 |
| **Ultimate Tensile kpsi** | 4500 | 4688 |

| **ASTM D638** | | |
|---|---|---|
| **Yield kpsi** | 3476 | 4082 |

5

| Elongation (break) % | 262 | 227 |

[values are averages of 5 tests]

ASTM D621 Creep Test

**Load**

| | | |
|---|---|---|
| 500 psi | .5 | .3 % deformation |
| 1000 psi | 1.6 | .7 |
| 2000 psi | 5.9 | 2.4 |

Additional evidence of the products' distinctiveness is found in data produced by small angle X-ray testing. A truly characteristic small-angle X-ray scattering plot of desmeared intensity (by the method of P. W. Schmidt, Acta Cryst., 13, 480 (1960) and Acta Cryst., 19, 938 (1965)) (I x (2 theta) squared) versus scattering angle (2 theta) for the material of the invention exhibits two distinct scattering peaks associated with crystal long-spacings in the range of 480 angstroms (at 2 theta = .184 degrees) and 4610 angstroms (at 2 theta = .0192 degrees). The presence of the sharp diffraction peak at the lower angle is indicative of an extended polymer chain conformation (with a lamellar thickness greater than 2000 angstroms) whereas the more diffuse higher-angle peak corresponds to a lamellar thickness characteristic of conventional folded chain PE. This provides clear evidence for the presence of two scattering peaks in the subject invention material which correspond to lamellar thicknesses both above and below 2000 angstroms. By comparison, the previously patented extended chain polyethylene of Lupton et al., was reported to exhibit a complete absence of any detectable small angle X-ray scattering in the range of 50 to 2000 angstroms. Consequently this work demonstrates that the subject invention material is morphologically distinguishable from Lupton et al.

EXAMPLE 2

The process described in Example 1 may be modified to yield a product with properties even more suitable for orthopedic replacements than the starting material. It is suggested that the UHMW polyethylene be preliminarily heated to a point closely approaching, but not reaching, the decomposition point of the UHMW polyethylene, preferably between 320-340°C, in an atmosphere of $N_2$ or in a vacuum for six hours. Once so pre-heated, the article is otherwise to be treated as in Example 1.

It is expected that the addition of the preliminary heating step to the process will yield a product displaying improved tensile yield strength, improved elongation (%) at break, and lower creep resistance than the product of Example 1 or the starting material.

EXAMPLE 3

Effect of Sequence of Heat-Treatment, Cooling, Reheating to a Lower Temperature, and Pressure Recrystallization on UHMWPE.

The process described in Example 2 may also be modified to yield a product with properties superior to that found in the starting material. It is suggested that the UHMW polyethylene be preliminarily heated to a point approaching, but not reaching, the decomposition point of the UHMW polyethylene, preferably between 320-340°C, in an atmosphere of $N_2$ or in a vacuum for 5 hours. It is then reheated to approximately 225°C, and pressure recrystallized as in Example 1.

It is expected that the described sequence of preliminary heat treatment, cooling, reheating to a lower temperature, and pressure recrystallization will yield a product displaying improved elongation (%) at break, higher crystallinity index (IR), a higher IZOD impact value, and lower creep resistance than the starting material.

EXAMPLE 4

Effect of Preheating by Refluxing

The process described in Example 3 may be further modified to yield a product with properties superior to that of the starting material and with at least an improved elongation (%) at break as compared to the products yielded by other embodiments of the invention.

It is suggested that a rod approximately 3" x 18" of UHMWPE (e.g., Hoechst, Hostalen GUR 415) be preliminarily heated in refluxing vapors of Krytox®-143AZ (E. I. du Pont de Nemours and Company, Wilmington, Delaware) at approximately 333-335°C for more than 0.5 hours.

Krytox®-143AZ is a perfluoroalkylpolyether that is a non-flammable, chemically inert liquid having unusually high thermal and oxidative stability. Other materials demonstrating these characteristics may also be suitable. The refluxing system should be protected by a nitrogen or other inert atmosphere and wrapped with glass insulation to facilitate slow, non-precipitous cooling.

It is expected that the described sequence of preliminary heat treatment by refluxing, cooling, reheating to a lower temperature, and pressure recrystallization will yield a product displaying improved elongation (%) at break, expected to be from 250-900, while retaining a high tensile strength at yield and a high tensile modulus.

EXAMPLE 5

A 3" diameter bar (rod), 18" in length, of American Hoechst Hostalen GUR 415 ultrahigh molecular weight polyethylene, would be heated in an oven and then would be encapsulated with low molecular weight polyethylene by rolling the hot rod onto a 1/16" sheet of low molecular weight polyethylene heated to 180°C on a large hot plate. An intervening sheet of "Teflon" Polytetrofluoroethylene film should be kept on the encapsulated rod to prevent sticking to the hot plate. The rod ends are similarly sealed. The "Teflon" film should be kept on the encapsulated rod to prevent sticking in the reactor.

The bar should be heated to 225°C under a nitrogen atmosphere and transfered to the reactor at 225°C. After sealing, the reactor pressure is taken to 3000 atmospheres which should cause the temperature to reach 237°C. The reactor should be permitted to cool to 180°C in 6.5 h, then maintained at this temperature for 1h. The temperature is dropped to 170°C, held at this temperature for 3h, then should be cooled slowly to 150°C from where it is cooled rapidly.

The rod, which remains coated, should be cut and machined into two test pieces (A and B) which should give results showing improved properties. For example, one would expect to find at 1st Heat a melting point, °C, in the range of 149 to 155 and a heat of fusion, J/g, in the range of 200.0 to 220.0. At 2nd heat melting point, °C, is expected in the range of 130 to 140.0 with a heat of fusion, J/g, expected in the range of 140.0 to 146. At crystallinity index (IR) of approximately 0.57, the tensile strength of the material (psi) at yield is expected in the range of 4000 to 4500, at maximum is expected in the range of 7000 to 9000, and at break is expected in the range of 7000 to 9000. Elongation, % at break, is expected in the range of 320 to 350. Modulus, Kpsi, is expected in the range of 350 to 365.0. Creep Deformation, % measured by ASTM D621, is expected to be approximately 0.6 The IZOD Impact (ftlb/in. of notch) is expected to be in the range of 15.5 to 16.0.

EXAMPLE 6

A 5.75" segment of enhanced ultrahigh molecular weight polyethylene prepared as in Example 1, should be skived to two films (A and B), of 11 mil and 5 mil thickness, respectively. The following properties may be expected.

The tensile strength of the material (psi) at yield is expected to range from 3000 to 3200, at maximum is expected to range from 4000 to 7000, at break is expected to range from 4000 to 7000, and at 5% elongation is expected to be 2500 to 2800. The tensile modulus (kpsi) is expected ro range from 125.0 to 200.0. The elongation at break (%) is expected to range from 200 to 500.

The skived films could be hot drawn in a tenter frame at 140°C. If one piece of the 5 mil film is drawn 6 fold in one direction the results could be tensile strength (psi) at yield that is approximately 37,820, at maximum that is approximately 42,100, at break that is approximately 46,400. Tensile modulus (Kpsi) could be approximately 93. Elongation at break (%) could be approximately 56 with a thickness in mils of 2.6.

If a second piece of the 5 mil film could be drawn 3 fold in both directions the results could be tensile strength (psi) at yield that is approximately 13,800, at maximum that is approximately 19,400, at break that is approximately 19,000. Tensile modulus (Kpsi) could be approximately 95.0. Elongation at break (%) could be approximately 132 with a thickness in mils of 1.6.

Conversions :

| | |
|---|---|
| 1 p.s.i | = 6.9 kPa |
| 1 ft.lb/in | = 0.534 J/cm |
| 1 atmosphere | = 98.1 kPa |
| 1 inch (") | = 2.54 cm |
| 1 ft.lb/in$^2$ | = 0.210 J/cm$^2$ |
| 1 mil | = 25.4 $\mu$m |
| 1 Å | = $10^{-10}$ m |
| 1 ft (') | = 0.30 m |

**Claims**

1. A process for obtaining a shaped article of an ultrahigh molecular weight linear polyethylene exhibiting a flexural modulus of 250,000-500,000 psi, (1724 - 3448 Mpa) a tensile stress at yield of 3500-4500 psi (24.13 - 31.03 Mpa), a tensile stress at break of 4000-9000 psi (27.6 - 62.1 Mpa), a tensile modulus of 300,000-700,000 psi, (2069 - 4827 Mpa) an elongation of 200-500% a notched Izod impact resistance of 12-25 ft. lb. per inch (6.4 - 13.3J/cm) of notch, a creep at a compression of 1000 psi (6895 kpa) of less than 1% after 24 hours at a temperature of 23°C and a relative humidity of 50%, the polyethylene having a molecular weight of 400,000 - 10,000,000, a single crystalline melting point of greater than 144°C, the reduction in said melting point upon remelting being greater than 11°C and an infrared crystallinity index of at least about 0.45 consisting essentially of the following steps:

    (a) forming said article of an ultrahigh molecular weight linear polyethylene having a molecular weight of 400,000 - 10,000,000;

    (b) surrounding said article with an inert material that is collapsible and impermeable;

    (c) subjecting said surrounded article to a gas under pressure of at least 280 Mpa and a temperature of 190°C - 300°C;

    (d) maintaining the temperature from 190°C - 300°C and the pressure of at least 280 Mpa for at least 0.5 hour;

    (e) reducing the temperature to below 160°C - 170°C, while maintaining the pressure at least 280 Mpa, the rate of reduction in temperature being such that temperature gradients in the shaped article are substantially avoided; and

    (f) cooling to a temperature of about 130°C or below and releasing the pressure to approximately 100 kPa in a manner such that remelting of said article is prevented.

2. The process of Claim 1 subjecting said article before step (C) to heat treatment at an elevated temperature not exceeding the decomposition point of the article in an inert atmosphere for at least 0.5 hour.

3. The process of Claim 2 wherein said heat treatment is at a temperature not exceeding 355°C.

4. The process of Claim 3 wherein said heat treatment is at a temperature of 320°-355°C.

5. The process of any one of claims 2 to 4 wherein said heat treatment before step (C) is accomplished by reflux vapors.

6. The process of Claim 1 wherein said article is enclosed within a stainless steel material that prevents said gas from contacting the surfaces of said article.

7. The process of Claim 6 wherein said gas is argon.

8. The process of Claim 7 wherein said pressure in step (C) is at least 300 Mpa.

9. The process of Claim 7 wherein said temperature in step (c) is 190°C-230°C.

10. The process of Claim 7 wherein the temperature and pressure in step (f) is maintained for at least one hour.

11. A modification of the process of the process of Claim 1 wherein step (a) is performed after the performance of step (f).

**Patentansprüche**

1. Verfahren zur Gewinnung eines geformten Gegenstandes aus einem ultrahochmolekularen linearen Polyethylen, das einen Biegemodul von 250 000 bis 500 000 psi (1 724 bis 3 448 MPa), eine Streckspannung von 3 500 bis 4 500 psi (24,13 bis 31,03 MPa), eine Reißfestigkeit von 4 000 bis 9 000 psi (27,6 bis 62,1 MPa), einen Zugmodul von 300 000 bis 700 000 psi (2 069 bis 4 827 MPa), eine Dehnung von 200 bis 500 %, eine Izod-Kerbschlagzähigkeit von 12 bis 25 ft.lb. per inch (6,4 bis 13,3 J/cm) der Kerbe, ein Kriechen bei einer Kompression von 1 000 psi (6 895 kPa) von weniger als 1 % nach 24 h bei einer Temperatur von 23 °C und einer relativen Luftfeuchtigkeit von 50 %, wobei das Polyethylen ein Molekulargewicht von 400 000 bis 10 000 000 hat, einen einzigen kristallinen Schmelzpunkt oberhalb von 144 °C, wobei die Erniedrigung dieses Schmelzpunktes beim erneuten Schmelzen höher als 11 °C ist, und einen Infrarot-Kristallinitäts-Index von wenigstens etwa 0,45 zeigt, bestehend im wesentlichen aus den folgenden Schritten:
   (a) Bilden des betreffenden Gegenstandes aus einem ultrahochmolekularen linearen Polyethylen mit einem Molekulargewicht von 400 000 bis 10 000 000;
   (b) Umhüllen des Gegenstandes mit einem inerten Material, das zusammenlegbar und undurchlässig ist;
   (c) Einwirkenlassen eines Gases unter einem Druck von wenigstens 280 MPa und bei einer Temperatur von 190 °C bis 300 °C auf den umhüllten Gegenstand;
   (d) Aufrechterhalten der Temperatur von 190 °C bis 300 °C und des Druckes von wenigstens 280 MPa über wenigstens 0,5 h;
   (e) Senken der Temperatur auf unterhalb von 160 °C bis 170 °C und Aufrechterhalten des Druckes von wenigstens 280 MPa, wobei die Geschwindigkeit der Temperaturerniedrigung eine solche ist, daß Temperaturgradienten in dem geformten Gegenstand im wesentlichen vermieden werden; und
   (f) Kühlen auf eine Temperatur unterhalb von 130 °C oder darunter und Entspannen des Druckes auf etwa 100 kP in einer solchen Weise, daß ein erneutes Schmelzen des Gegenstandes verhindert wird.

2. Verfahren nach Anspruch 1, worin der Gegenstand vor dem Schritt (c) einer wenigstens 0,5 h dauernden Wärmebehandlung bei einer erhöhten Temperatur, die den Zersetzungspunkt des Gegenstandes nicht übersteigt, in einer inerten Atmosphäre unterworfen wird.

3. Verfahren nach Anspruch 2, worin die Wärmebehandlung bei einer Temperatur erfolgt, die 355 °C nicht übersteigt.

4. Verfahren nach Anspruch 3, worin die Wärmebehandlung bei einer Temperatur von 320 °C bis 355 °C erfolgt.

5. Verfahren nach irgendeinem der Ansprüche 2 bis 4, worin die Wärmebehandlung vor dem Schritt (c) durch Rückflußdämpfe erfolgt.

6. Verfahren nach Anspruch 1, worin der Gegenstand innerhalb eines nichtrostenden Stahl-Materials eingeschlossen wird, das verhindert, daß das Gas die Oberflächen des Gegenstandes berührt.

7. Verfahren nach Anspruch 6, worin das Gas Argon ist.

8. Verfahren nach Anspruch 7, worin der Druck in Schritt (c) wenigstens 300 MPa beträgt.

9. Verfahren nach Anspruch 7, worin die Temperatur in Schritt (c) 190 °C bis 230 °C beträgt.

**10.** Verfahren nach Anspruch 7, worin die Temperatur und der Druck in Schritt (f) wenigstens 1 h aufrechterhalten werden.

**11.** Modifikation des Verfahrens nach Anspruch 1, worin der Schritt (a) nach der Durchführung des Schrittes (f) durchgeführt wird.

**Revendications**

**1.** Procédé d'obtention d'un article façonné formé d'un polyéthylène linéaire de poids moléculaire très élevé, présentant un module de flexion de 1 724 à 3 448 MPa, une limite élastique de 24,13 à 31,03 MPa, une résistance à la rupture par traction de 27,6 à 62,1 MPa, un module en traction de 2 069 à 4 827 MPa, un allongement de 200 à 500 %, une résistance au choc sur barreau entaillé selon Izod de 6,4 à 13,3 J/cm d'entaille, une valeur de fluage, à une compression de 6 895 kPa, de moins de 1 % après 24 heures à la température de 23°C et à un taux d'humidité relative de 50 %, le polyéthylène ayant un poids moléculaire de 400 000 à 10 000 000, un unique point de fusion cristallin supérieur à 144°C, la diminution dudit point de fusion après une refonte étant supérieure à 11°C, et un indice de cristallinité à l'infrarouge d'au moins environ 0,45, comprenant essentiellement les étapes suivantes :
(a) former ledit article en polyéthylène linéaire de poids moléculaire très élevé, ayant un poids moléculaire de 400 000 à 10 000 000 ;
(b) entourer ledit article d'un matériau inerte qui est compressible et imperméable ;
(c) soumettre ledit article entouré à l'action d'un gaz, à une pression d'au moins 280 MPa et une température de 190°C à 300°C ;
(d) maintenir la température de 190 à 300°C et la pression d'au moins 280 MPa pendant au moins 0,5 heure ;
(e) abaisser la température au-dessous de 160 à 170°C tout en maintenant la pression à au moins 280 MPa, la vitesse de diminution de la température étant telle que des gradients de température dans l'article façonné sont essentiellement évités ; et
(f) refroidir l'article à une température d'environ 130°C ou moins et relâcher la pression à environ 100 kPa d'une manière telle que la refonte dudit article est empêchée.

**2.** Procédé de la revendication 1, dans lequel on soumet ledit article, avant l'étape (c), à un traitement thermique à une température élevée ne dépassant pas le point de décomposition de l'article, dans une atmosphère inerte, pendant au moins 0,5 heure.

**3.** Procédé de la revendication 2, dans lequel on effectue ledit traitement thermique à une température ne dépassant pas 355°C.

**4.** Procédé de la revendication 3, dans lequel on effectue ledit traitement thermique à une température de 320 à 355°C.

**5.** Procédé selon l'une quelconque des revendications 2 à 4, dans lequel on effectue ledit traitement thermique, avant l'étape (c), au moyen de vapeurs au reflux.

**6.** Procédé de la revendication 1, dans lequel ledit article est enfermé à l'intérieur d'un matériau en acier inoxydable qui empêche ledit gaz de venir au contact des surfaces dudit article.

**7.** Procédé de la revendication 6, dans lequel ledit gaz est l'argon.

**8.** Procédé de la revendication 7, dans lequel ladite pression utilisée lors de l'étape (c) est d'au moins 300 MPa.

**9.** Procédé de la revendication 7, dans lequel ladite température utilisée lors de l'étape (c) est de 190 à 230°C.

**10.** Procédé de la revendication 7, dans lequel on maintient la température et la pression utilisées lors de l'étape (c) pendant au moins une heure.

11. Modification du procédé de la revendication 1, dans lequel on effectue l'étape (a) après avoir effectué l'étape (f).